# EUROPÄISCHE PATENTSCHRIFT

(11) **EP 1 508 326 B1**
(45) Veröffentlichungstag und Bekanntmachung des Hinweises auf die Patenterteilung: **28.03.2007**
(21) Anmeldenummer: 04103844.9
(22) Anmeldetag: 10.08.2004
(51) Int. Cl.: A61K 8/49, A61Q 17/04

(54) **Verwendung von UV-Filtersubstanzen zur Optimierung der Qualität von kosmetischen Schäumen**
Use of UV sunscreen agents to optimize the quality of cosmetic foams
Utilisation de filtres UV pour optimiser la qualité des mousses cosmétiques

(30) Priorität: 19.08.2003 DE 10338012
(43) Veröffentlichungstag der Anmeldung: 23.02.2005
(73) Patentinhaber: Beiersdorf AG, 20245 Hamburg (DE)
(72) Erfinder: Eitrich, Anja, 22587, Hamburg (DE); Riedel, Heidi, 22529, Hamburg (DE); Mundt, Claudia, 28207, Bremen (DE); Von Bülow, Rixa, 20255, Hamburg (DE); Oelrichs, Ilka, 25436, Tornesch (DE); Hoop, Kerstin, 25469, Halstenbeck (DE); Brunckhorst, Melanie, 20535, Hamburg (DE); Scheede, Stefan, 20259, Hamburg (DE)

(56) Entgegenhaltungen:
- EP-A- 1 388 338
- WO-A-20/04022020

## Beschreibung

Die vorliegende Erfindung betrifft die Verwendung von UV-Filtersubstanzen zur Optimierung der Qualität von selbstschäumenden, schaumförmigen, nachschäumenden oder schäumbaren kosmetischen und dermatologischen Zubereitungen.

Schäume bzw. schaumförmige Zubereitungen gehören zu den dispersen Systemen.

Das bei weitem wichtigste und bekannteste disperse System stellen Emulsionen dar. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rü h-ren der Flüssigkeit in der betreffenden Gasatmosphäre, vorausgesetzt, daß die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, daß sie eine feine Verteilung von Wirkstoffen auf der Haut erlauben. Allerdings sind kosmetische Schäume in der Regel nur durch Verwendung besonderer Tenside, welche darüberhinaus oft wenig hautverträglich sind, zu erreichen.

Ein Nachteil des Standes der Technik ist es, daß derartige Schäume nur wenig stabil sind, weshalb sie üblicherweise innerhalb von etwa 24 Stunden zusammenfallen. Eine Anforderung an kosmetische Zubereitungen ist aber, daß diese eine möglichst jahrelange Stabilität besitzen. Diesem Problem wird im allgemeinen dadurch Rechnung getragen, daß der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterläßt einen Schaum.

Auch nachschäumende kosmetische Zubereitungen sind an sich bekannt. Sie werden zunächst in fließförmiger Form aus einem Aerosolbehälter auf die Haut aufgetragen und entwickeln nach kurzer Verzögerung erst dort unter dem Einfluß des enthaltenen Nachschäummittels den eigentlichen Schaum, beispielsweise einen Rasierschaum. Nachschäumende Zubereitungen liegen oft in speziellen Ausführungsformen wie etwa nachschäumenden Rasiergelen oder dergleichen vor.

Der Stand der Technik kennt ferner kosmetische oder dermatologische Zubereitungen, welche bereits bei der Herstellung aufgeschäumt werden und dabei an sich eine genügend hohe Stabilität aufweisen, um in üblicher Weise verpackt, gelagert und in den Handel gebracht zu werden (siehe beispielsweise DE-1 00 63 340-A1, DE-1 00 63 341-A1 und DE-100 63 342-A1).

Ein Nachteil der Zubereitungen des Standes der Technik ist allerdings häufig die Qualität des Schaumes, d. h. der Schaum ist weich, grobblasig und nach Entnahme aus dem Behältnis nur wenig stabil. Auch schäumbare kosmetische Emulsionen (sog. "Aerosolschäume") lassen sich nach dem Stand der Technik ohne die Verwendung besonderer Tenside nicht formulieren bzw. technisch herstellen. Dieses gilt insbesondere für Systeme, die auf klassischen Emulgatoren basieren. Nach dem Stand der Technik entwickeln derartige Systeme unter Zusatz von Treibgas ausschließlich wäßrig-feuchte Schäume, die nach Applikation schnell brechen.

Nach dem Patentdokument WO 2004/022020 verbessert der Zusatz des UV-Filters 2-(4-Diethylamino -2-hydroacybenzoyl) -benzoesäure-n-hexylester die Schaumstabilität.

Eine Aufgabe der vorliegenden Erfindung war also, den Stand der Technik zu bereichern und kosmetische oder dermatologische selbstschäumende und/oder schaumförmige Zubereitungen zur Verfügung zu stellen, die die Nachteile des Standes der Technik nicht aufweisen.

Es war überraschend und für den Fachmann nicht vorauszusehen, daß
die Verwendung einer oder mehrerer UV-Filtersubstanzen zur Schaumverstärkung selbstschäumender, schaumförmiger, nachschäumender oder schäumbarer kosmetischer und dermatologischer Zubereitungen
den Nachteilen des Standes der Technik abhelfen.

Unter "selbstschäumend", "schaumförmig", "nachschäumend" bzw. "schäumbar" ist im Sinne der vorliegenden Erfindung zu verstehen, daß die Gasbläschen (beliebig) verteilt in einer (oder mehreren) flüssigen Phase(n) vorliegen, wobei die Zubereitungen makroskopisch nicht notwendigerweise das Aussehen eines Schaumes haben müssen. Kosmetische oder dermatologische Zubereitungen im Sinne der vorliegenden Erfindung (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind Schäume gemäß der vorliegenden Erfindung - insbesondere dann, wenn die Gasbläschen zu klein sind, um unter einem Lichtmikroskop erkannt zu werden - auch an der starken Volumenzunahme des Systems erkennbar.

Durch die Erfindung wird erstmalig ein gehaltvoller, kompakter Cremeschaum ("Mousse") zugänglich, der sich über eine lange Lagerdauer sowie durch eine außerordentlich hohe Stabilität und ein kompaktes Erscheinungsbild auszeichnet. Erfindungsgemäße Zusammensetzungen entwickeln sich bereits während ihrer Herstellung - beispielsweise während des Rührens oder bei der Homogenisierung - zu feinblasigen Schäumen. Erfindungsgemäß sind feinblasige, reichhaltige Schäume von hervorragender kosmetischer Eleganz erhältlich. Weiterhin sind erfindungsgemäß besonders gut hautverträgliche Zubereitungen erhältlich, wobei wertvolle Inhaltsstoffe besonders gut auf der Haut verteilt werden können.

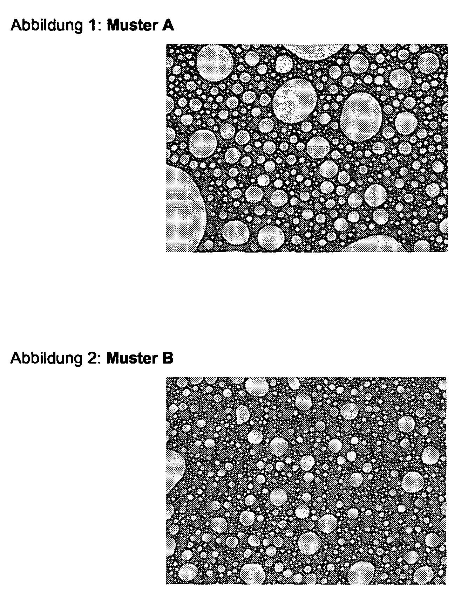

Die Qualität von Schäumen, welche polare Ölen enthalten, ist gegenüber der von Schäumen, welche nur unpolare Öle enthalten, schlechter: Der Schaum ist weicher, grobblasiger und weniger stabil (vergleiche Abbildung 1).

Überraschenderweise ist die Schaumqualität von Schäumen enthaltend polare Öle deutlich besser, wenn zusätzlich zu dem polaren Öl noch mindestens eine UV-Filtersubstanz eingesetzt wird: Der Schaum wird kompakt, feinblasig, stabil und cremig (siehe Abbildung 2).

Gegenstand der Erfindung ist daher ferner die
die Verwendung einer oder mehrerer UV-Filtersubstanzen nach Anspruch 1 zur Schaumverstärkung selbstschäumender, schaumförmiger, nachschäumender oder schäumbarer kosmetischer und dermatologischer Zubereitungen, welche mindestens eine polare Ölkomponente enthalten.

Mit "Schaumverstärkung" ist darüberhinaus gemeint, daß die Stabilität der aufgeschäumten Zubereitungen (die "Schaumstabilität") gegenüber ansonsten gleichen Zubereitungen, welche keine erfindungsgemäßen UV-Filtersubstanzen enthalten, deutlich verbessert wird, d. h. durch die erfindungsgemäße Verwendung wird ein Brechen der Schäume zeitlich verzögert.

Ferner ist unter "Schaumverstärkung" im Sinne der vorliegenden Erfindung zu verstehen, daß auch die kosmetischen Eigenschaften der erfindungsgemäßen Schäume im Vergleich zu Zubereitungen, welche keine erfindungsgemäßen UV-Filtersubstanzen enthalten, deutlich verbessert werden: So erhält man durch die erfindungsgemäße Verwendung reichhaltige, feste Schäume ("Schaum-Cremes"), welche trotz ihrer Kompaktheit und Reichhaltigkeit leicht verteilbar sind und schnell einziehen.

Insbesondere ist unter "Schaumverstärkung" im Sinne der vorliegenden Erfindung zu verstehen, daß die Blasengröße der erfindungsgemäßen Schäume im Vergleich zu Zubereitungen, welche keine erfindungsgemäßen UV-Filtersubstanzen enthalten, kleiner ist. Demgegenüber ist die Anzahl die Blasen im gleichen Volumen erhöht.

Mit "Blasen" sind im Sinne der vorliegenden Erfindung die Gasbläschen gemeint, unter Blasengröße" ist dementsprechend der Durchmesser der Gasbläschen zu verstehen.

Dieser Effekt kann sehr gut anhand einer mikroskopischen Schaumuntersuchung verdeutlicht werden. Ziel dieser Untersuchung ist die mikroskopische Bestimmung der Anzahl und der Größe der Blasen in kosmetischen Produkten.

Eine mikroskopischen Schaumuntersuchung läßt sich beispielsweise wie folgt bei einer konstanten Temperatur von 21 °C durchführen:
- Von der zu untersuchenden kosmetischen Zubereitung wird an drei verschiedenen Stellen (z. B. oben, Mitte, unten) aus dem Behälter jeweils eine Probe mittels Spatel entnommen (entsprechend drei Proben pro Zubereitung). (Aerosolschäume werden in ein Reservoir oder auf Zellstoff gesprüht und die Proben aus dem Reservoir gezogen.)
- Einwaage von 9 mg ± 0,5mg auf Objektträgern
- Abdeckung der Probe mit einem Abdeckglas
- Vor der eigentlichen Aufnahme wird auf den Objektträger mit Abdeckglas für 15 sec ein Gewicht von 20 g gelegt.
- Anschließend werden von jeder Probe drei Bilder aufgenommen, wobei die untersuchte Fläche 6,22 mm x 4,66 mm = 28,11 mm² beträgt. Dazu wird das Mikroskop auf 125fache Vergrößerung eingestellt und die Probe im Durchlicht beleuchtet (Digitalkamera Polaroid DMC und Bildanalyse-Programm KS400).

Vorteilhafte Zubereitungen im Sinne der vorliegenden Erfindung haben eine mittlere Blasengröße von 0,005 bis 0,5 mm², vorteilhaft von 0,005 bis 0,2 mm², besonders voteilhaft von 0,005 bis 0,1 mm² sowie eine Blasenanzahl von 100 bis 4000, vorteilhaft von 500 bis 3000, besonders vorteilhaft von 1000 bis 3000, jeweils bezogen auf den untersuchten Bildausschnitt (s.o.).

Vorteilhafte UV-Filtersubstanzen im Sinne der vorliegenden Erfindung sind die im folgenden genannten, welche in der Wasser- und/oder der Ölphase vorliegen können.

Die erfindungsgemäßen Zubereitungen können vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten.

Besonders vorteilhafte bei Raumtemperatur flüssige UV-Filtersubstanzen im Sinne der vorliegenden Erfindung sind, 2 -Ethylhexyl-2-cyano-3,3-diphenylacrylat (INCI: Octocrylene) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan / Dimethylsiloxan - Copolymer welches beispielsweise unter der Handelsbezeichnung Parsol® SLX bei Hoffmann La Roche erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen im Sinne der vorliegenden Erfindung sind Hydroxybenzophenone, welche sich durch die folgende Strukturformel auszeichnen: 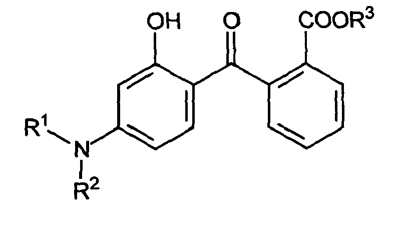 worin
R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
R³ einen C₁-C₂₀-Alkyl Rest bedeutet.

Ein besonders vorteilhaftes Hydroxybenzophenon im Sinne der vorliegenden Erfindung ist das 2-(4'-Diethylamino-2'-hydoxybenzoyl)-benzoesäurehexylester (auch: Aminobenzophenon), welches sich durch folgende Struktur auszeichnet: 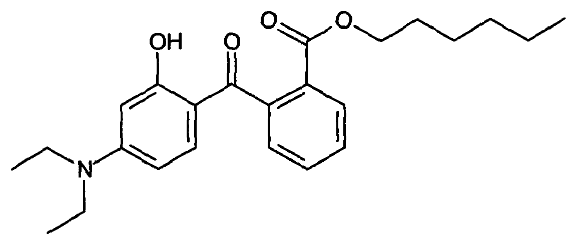 und unter dem Uvinul A Plus bei der Fa. BASF erhältlich ist.

Erfindungsgemäß bevorzugt ist ferner das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 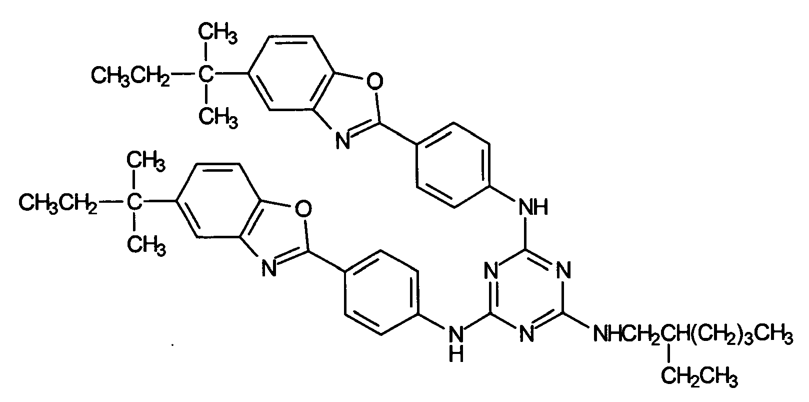 auszeichnet und bei 3V Sigma unter der Handelsbezeichnung Uvasorb® K2A erhältlich ist.

Das 2,4-bis-[5-1(dimethylpropyl)benzoxazol-2-yl-(4-phenyl)-imino]-6-(2-ethylhexyl)-imino-1,3,5-triazin liegt vorteilhaft in gelöster Form in den erfindungsgemäßen kosmetischen Zubereitungen vor. Es kann ggf. aber auch von Vorteil sein, wenn das 2,4-bis-[5-1(dimethylpropyl)benzoxazol-2-yl-(4-phenyl)-imino]-6-(2-ethylhexyl)-imino-1,3,5-triazin in pigmentärer, d. h. ungelöster Form - bei spielsweise in Partikelgrößen von 10 nm bis zu 300 nm - vorliegt.

Die Gesamtmenge an 2,4-bis-[5-1(dimethylpropyl)benzoxazol-2-yl-(4-phenyl)-imino]-6-(2-ethylhexyl)-imino-1,3,5-triazin in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylenerbis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch Bis-Resorcinyltriazinderivate sind vorteilhafte UV-Filtersubstanzen im Sinne der vorliegenden Erfindung. Sie zeichnen sich durch die folgende Strukturformel aus: 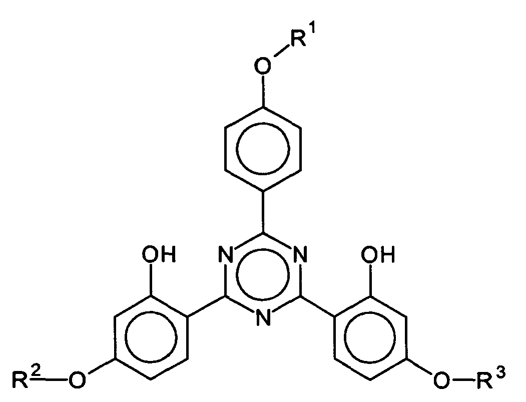 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Erfindungsgemäß besonders bevorzugt ist das 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist. Ein weiteres bevorzugtes Bis-Resorcinyltriazinderivat ist das 2-[4,6-Bis(2,4-dimethylphenyl)-1,3,5-triazin-2-yl]-5-(octyloxy)phenol (CAS Nr.: 2725-22-6).

Die Gesamtmenge an einem oder mehreren Bis-Resorcinyltriazinderivaten in den fertigen kosmetischen oder dermatologischen Zubereitungen wird vorteil haft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Besonders vorteilhafte UV-Filtersubstanzen sind:
- Phenylen-1,4-bis-(2-benzimidazyl)-3,3'-5,5'-tetrasulfonsäure-bis-natriumsalz (INCI: Bisimidazylate, Handelsname: Neoheliopan AP),
- Dioctylbutylamidotriazon (INCI: Diethylhexyl Butamido Triazone, Handelsname: Uvasorb HEB),
- 2,4-bis-[5-1(dimethylpropyl)benzoxazol-2-yl-(4-phenyl)-imino]-6-(2-ethylhexyl)-imino-1,3,5-triazin (Handelsname: Uvasorb K2A),
- 2-(4'-Diethylamino-2'-hydoxybenzoyl)-benzoesäurehexylester (auch: Aminobenzophenon) (INCI: Diethylamino Hydroxybenzoyl Hexyl Benzoat, Handelsname: Uvinul A plus),
- (3Z)-1,7,7-trimethyl-3-(4-methylbenzylidene)bicyclo[2.2.1]heptan-2-one (INCI: 4-Methylbenzylidene Campher, Handelsname: Eusolex 6300),
- 2-Ethylhexyl-2-cyano-3,3-diphenylacrylat (INCI: Octocrylene, Handelsname: Uvinul N-539),
- Benzol-1,4-di(2-oxo-3-bornylidenmethyl-10-sulfonsäure) (INCI: Terephtalidene Dicampher Sulfonsäure, Handelsname: Mexoryl SX),
- 2-(2H-benzotriazol-2-yl)-4-methyl-6-[2-methyl-3-[1,3,3,3-tetramethyl-1-[(trimethylsilyl)oxy]disiloxanyl]propyl]-phenol (INCI: Drometrizole Trisiloxane, Handelsname: Mexoryl XL).

Ganz besonders vorteilhafte UV-Filtersubstanzen im Sinne der vorliegenden Erfindung sind:
- 2,4,6-Tris-[anilino-(p-carbo-2'-ethyl-1'-hexyloxy)]-1,3,5-triazin (INCI: Octyl Triazon, Handelsname: Uvinul T150),
- 2-Ethylhexyl-4-methoxycinnamat (INCI: Octyl Methoxycinnamat, Handelsname: Parsol MCX),
- 4-(tert.-Butyl)-4'-methoxydibenzoylmethan (INCI: Butylmethoxydibenzoylmethan, Handelsname: Parsol 1789),
- 2,4-Bis-{[4-(2-Ethyl-hexyloxy)2-hydroxy]-phenyl}-6-(4-methoxyphenyl)-1,3,5-triazin (INCI: Bis-Ethylhexyloxylphenol Methoxyphenyl Triazin, Handelsname: Tinosorb S),
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (Handelsname: Eusolex 232).

Vorteilhaft enthalten die Zubereitungen im Sinne der vorliegenden Erfindung die die UV-Filtersubstanzen in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen.

Besonders vorteilhafte Zubereitungen im Sinne der vorliegenden Erfindung enthalten ein Emulgatorsystem, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1:1:1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureestern des Sorbitans.

Die Gesamtmenge der weiteren Emulgatoren wird erfindungsgemäß vorteilhaft kleiner als 5 Gew.-%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte Zubereitungen im Sinne der vorliegenden Erfindung sind frei von Mono- oder Diglycerylfettsäureestern. Insbesondere bevorzugt sind erfindungsgemäße Zubereitungen, welche kein Glycerylstearat, Glycerylisostearat, Glyceryldi isostearat, Glyceryloleat, Glycerylpalmitat, Glyceryl myristat, Glyceryllanolat und/oder Glyceryllaurat enthalten.

Die Ölphase der erfindungsgemäßen Zubereitungen wird vorteilhaft gewählt aus der Gruppe der polaren Lipide mit einer Polarität ≤ 35 mN/m Besonders vorteilhafte Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Hersteller** | **Handelsname** | **INCl-Name** | **Polarität** |
|---|---|---|---|
| | | | **mN/m** |
| Cognis | Cetiol OE | Dicaprylylcarbonat | 31,7 |
| Cognis | Cetiol CC | Dicaprylylether | 30,9 |
| Stearinerie Dubois Fils | DUB VCI 10 | Isodecyl Neopentanoate | 29,9 |
| Lipo Chemicals Inc | Liponate TDTM | Tridecyl Trimellitate | 27,2 |
| Wacker | Wacker AK 100 | Dimethicone | 26,9 |
| Henkel Cognis | Eutanol G | Octyldodecanol | 24,8 |
| | Macadamia Nut Oil | | 22,1 |
| Bayer AG, Dow Coming | Silikonöl VP 1120 | Phenyl Trimethicone | 22,7 |
| Henkel Cognis | Isopropylstearat | Isopropyl Stearate | 21,9 |
| WITCO, Goldschmidt | Finsolv TN | C12-15 Alkyl Benzoate | 21,8 |
| Dr. Straetmans | Dermofeel BGC | Butylene Glycol Dicaprylate/Dicaprate | 21,5 |
| Unichema Huels | Miglyol 812 | CapryliclCapric Triglyceride | 21,3 |
| Henkel Cognis | Cetiol B | Dibutyl Adipate | 14,3 |
| Condea Augusta S.P A | Cosmacol ELI | C12-13 Alkyl Lactate | 8,8 |
| Condea Augusta S.P.A. | Cosmacol ETI | Di-C12/13 Alkyl Tartrate | 7,1 |
| Henkel Cognis | Myritol 331 | Cocoglycerides | 5,1 |
| Symrise | Corapan TQ | Diethylhexylnaphthalat | n.b. |

Es ist ganz besonders vorteilhaft im Sinne der vorliegenden Erfindung, eine oder mehrere UV-Filtersubstanzen zur Schaumverstärkung selbstschäumender, schaumförmiger, nachschäumender oder schäumbarer kosmetischer und dermatologischer Zubereitungen zu verwenden, welche Butylene Glycol Caprylate/Caprate, C12-15 Alkyl Benzoate und/oder Di-C12/13 Alkyl Tartrate enthalten.

Von den Kohlenwasserstoffen sind insbesondere Paraffinöl sowie weitere hydrierte Polyolefine wie hydriertes Polyisobutene, Squalan und Squalen vorteilhaft im Sinne der vorliegenden Erfindung zu verwenden.

Die Gehalt der Lipide wird vorteilhaft kleiner als 50 Gew.-% gewählt, bevorzugt zwischen 1 und 40 Gew.-%, insbesondere bevorzugt zwischen 5 und 15 Gew.-%, jeweils bezogen auf das Gesamtgewicht der schäumbaren Zubereitung.

Falls die Lipidphase öllösliche UV-Filtersubstanzen enthält, ist es vorteilhaft den Gehalt der Lipidphase kleiner als 80 Gew.-% zu wählen, bevorzugt zwischen 1 und 40 Gew.-%, insbesondere bevorzugt zwischen 5 und 30 Gew.-%, jeweils bezogen auf das Gesamtgewicht der schäumbaren Zubereitung.

Es kann gegebenenfalls vorteilhaft sein, wenngleich es nicht zwingend ist, wenn die Ölphase der Zubereitungen im Sinne der vorliegenden Erfindung auch unpolare Lipide enthält.

Die erfindungsgemäßen kosmetischen und/oder dermatologischen Zubereitungen können wie üblich zusammengesetzt sein. Besonders vorteilhaft im Sinne der vorliegenden Erfindung sind Zubereitungen zur Pflege der Haut: sie können dem kosmetischen und/oder dermatologischen Lichtschutz, ferner zur Behandlung der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische oder topische dermatologische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Ebenso wie Emulsionen von flüssiger und fester Konsistenz als kosmetische Reinigungslotionen bzw. Reinigungscremes Verwendung finden, können auch die erfindungsgemäßen Zubereitungen "Reinigungsschäume" darstellen, welche beispielsweise zum Entfernen von Schminken und/oder Make-up oder als milder Waschschaum - ggf. auch für unreine Haut - verwendet werden können. Derartige Reinigungsschäume können vorteilhaft ferner als sogenannte rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die kosmetischen oder dermatologischen Mittel gemäß der Erfindung können beispielsweise aus Aerosolbehältern entnommen und dabei aufgeschäumt werden. Erfindungsgemäße Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Deratige Behälter können mit Ventilen sehr unterschiedlicher Bauart ausgestattet sein, die die Entnahme des Inhalts als Schaum ermöglichen.

Ferner vorteilhaft können die Zubereitungen gemäß der vorliegenden Erfindung aus Treibgas-freien, mechanisch zu bedienenden Pumpzerstäubem (Pumpspendern) entnommen werden. Insbesondere vorteilhaft im Sinne der vorliegenden Erfindung sind Pumpsysteme, welche ohne Druckgas, aber mit einem Filter, der spezielle Verwirbelungen bewirkt, arbeiten.

Die kosmetischen oder dermatologischen Zubereitungen gemäß der Erfindung können ferner vorzugsweise beispielsweise aus Zweikammeraerosolbehältern entnommen und auf die Haut aufgetragen werden. Erfindungsgemäß vorteilhafte Packmittel sind Behältnisse, in denen sich eine Kammer mit einer Füllung aus den flüssigen bzw. breiartigen Zubereitungen unter dem Druck eines in einer zweiten Kammer befindlichen stehenden Primärtreibmittels befindet. Derartige Behälter können mit Ventilen sehr unterschiedlicher Bauart ausgestattet sein, die die Entnahme des Inhalts der ersten Kammer als Emulsion oder Gel in jeder Lage - auch mit dem Ventil nach unten - ermöglichen. Eine vorteilhafte Ausführungsform sind BiCan^{®}-Aerosolbehälter, bei denen das Produkt in einem flexiblen Beutel aus Metall oder Kunststoff innerhalb der Dose eingeschlossen ist.

Erfindungsgemäße nachschäumende Zusammensetzungen stellen ungeschäumt, also unmittelbar nach dem Austreten aus einem Aerosolbehälter, Zwei- oder Mehrphasensysteme - in der Regel Emulsionen - dar. Sie können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.

Es hat sich darüber hinaus in überraschender Weise herausgestellt, daß bei der Verwendung von (Sekundär-) Treibmitteln, besonders vorteilhaft von in der gegebenenfalls vorhandenen Ölphase löslichen Treibmitteln, also beispielsweise üblichen Propan-Butan-Gemischen, die erfindungsgemäßen Zubereitungen nicht einfach als Aerosoltröpfchen versprüht werden, sondern sich zu feinblasigen, reichhaltigen Schäumen entwickeln, sobald solche mit solchen (Sekundär-) Treibmitteln beladenen Systeme Druckentspannung erfahren.

Bei Verwendung von Kohlenwasserstoffen oder deren Gemischen mit 4 oder 5 Kohlenstoffatomen, insbesondere Isobutan, n-Pentan und Isopentan, als (Sekundär-) Treibmittel kann man das selbständige Aufschäumen nach dem Austritt aus der Druckverpackung zeitlich verzögern.

Durch das Verdampfen des Sekundärtreibmittels im applizierten Kosmetikprodukt wird der Haut ferner Wärme entzogen und ein angenehmer Kühleffekt erlangt. Solche nachschäumenden Zubereitungen werden daher ebenfalls als vorteilhafte Verkörperungen der vorliegenden Erfindung mit eigenständiger erfinderischer Tätigkeit angesehen.

Als Druckgasbehälter kommen im Sinne der vorliegenden Erfindung vor allem zylindrische Gefäße aus Metall (Aluminium, Weißblech, Inhalt <1000 mL), geschütztem bzw. nicht-splitterndem Glas oder Kunststoff (Inhalt < 220 mL) bzw. splitterndem Glas oder Kunststoff (Inhalt < 150 mL) in Frage, bei deren Auswahl Druck- und Bruchfestigkeit, Korrosionsbeständigkeit, leichte Füllbarkeit, ggf. Sterilisierbarkeit usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 12 bar und das maximale Füllvolumen bei dieser Temperatur ca. 90 % des Gesamtvolumens. Für Glas- und Kunststoffdosen gelten niedrigere, von der Behältergröße und dem Treibmittel (ob verflüssigtes, verdichtetes oder gelöstes Gas) abhängige Werte für den Betriebsdruck.

Besonders vorteilhaft im Sinne der vorliegenden Erfindung sind Dosen aus Weißblech, Aluminium und Glas. Aus Korrosionsschutzgründen können Metalldosen innen lackiert sein (silber- oder goldlackiert), wozu alle handelsüblichen Innenschutzlacke geeignet sind. Bevorzugt im Sinne der vorliegenden Erfindung sind Polyester-, Epoxyphenol- sowie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt. Werden Kunststoffe als Sprüh-Behältermaterial verwendet, so sollten diese Chemikalien- und Sterilisationstemperatur-beständig, gasdicht, schlagfest und gegen Innendrücke über 12 bar stabil sein. Prinzipiell für SprühBehälter-Zwecke geeignet sind Polyacetale und Polyamide.

Der innere Aufbau der Sprüh-Dosen sowie die Ventilkonstruktion sind je nach Verwendungs-Zweck und der physikalischen Beschaffenheit des Inhalts - z. B. ob als Zwei- oder als Dreiphasensystem - sehr variantenreich und können vom Fachmann durch einfaches Ausprobieren ohnen erfinderisches Zutun ermittelt werden. Für geeignete Ausführungsformen sei auf das "Aerosol Technologie Handbuch der Aersosol-Verpackung" hingewiesen (*Wolfgang Tauscher, Melcher Verlag GmbH Heidelberg*/*München, 1996*).

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:
- Teller:: Weißblech: blank, gold- bzw. klarlackiert, folienkaschiert (PE, PP oder PET) Aluminium: blank, silber- oder goldlackiert, verschiedene Lackvarianten, Stoner-Mudge-Ausführung
- Dichtung:: natürliche bzw. synthetische Elastomere bzw. thermoplastische (Sleeve-Gaskets, folienkaschiert aus PE oder PP) Innen- und Aussendichtungen, z. B. aus Perbunan, Buna, Neopren, Butyl, CLB, LDPE, Viton, EPDM, Chlorbutyl, Brombutyl und/oder diversen Compounds
- Kegel:: PA, POM, Messing sowie diversen Sondermaterialen, Standardbohrungen (z. B.: 0,25 bis 0,70 mm oder 2 x 0,45 bis 2 x 1,00 mm), verschiedene Schaftdurchmesser
- Feder:: Metall, besonders bevorzugt V2A, rostfreier Stahl; Kunststoff und auch Elastomer
- Gehäuse:: Standard und Impact
VPH-Bohrungen, RPT-Bohrungen oder geschlitzt für Überkopf-Anwendungen
Materialien: z. B. Polyacetal, PA, PE, POM und dergleichen mehr
- Steigrohr:: Kunststoff (Polymer Resin), z.B. PE, PP, PA oder Polycarbonat

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Als Treibmittel sind die üblichen "klassischen" leichtflüchtigen, verflüssigten Treibgase, wie beispielsweise Dimethylether (DME) und/oder lineare oder verzweigtkettige Kohlenwasserstoffe mit zwei bis fünf Kohlenstoffatomen (wie insbesondere Ethan, Propan, Butan, Isobutan und/oder Pentan) geeignet, die allein oder in Mischung miteinander eingesetzt werden können.

Auch Druckluft sowie weitere unter Druck befindliche Gase wie Luft, Sauerstoff, Stickstoff, Wasserstoff, Helium, Krypton, Xenon, Radon, Argon, Lachgas (N₂O) und Kohlendioxid (CO₂) sind vorteilhaft im Sinne der vorliegenden Erfindung als Treibgase (sowohl einzeln als in beliebigen Mischungen miteinander) zu verwenden.

Natürlich weiß der Fachmann, daß es weitere an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere halogenierte (mit Fluor, Clor, Brom, lod und/oder Astat substituierte) Kohlenwasserstoffe wie beispielsweise Fluorkohlenwasserstoffe und Fluorchlorkohlenwasserstoffe (FCKW).

Die genannten Gase können im Sinne der vorliegenden Erfindung jeweils einzeln oder in beliebigen Mischungen zueinander verwendet werden.

Vorteilhaft im Sinne der vorliegenden Erfindung wird der Volumenanteil an Treibgas aus dem Bereich von 0,1 bis 30 Vol.-%, bezogen auf das Gesamtvolumen aus Füllgut und Treibgas gewählt (entsprechend einem Volumenanteil von 70 bis 99,9 Vol.-% Füllgut).

Besonders bevorzugtes Treibgas im Sinne der vorliegenden Erfindung ist Kohlendioxid. Insbesondere vorteilhaft sind aus erfindungsgemäßen Zubereitungen erhältliche Schäume, welche Kohlendioxid als einen oder den Wirkstoff enthalten.

Besonders vorteilhafte, feincremige und reichhaltige Schäume sind erhältlich, wenn die erfindungsgemäßen Zubereitungen mit Hilfe von linearen oder verzweigtkettigen, halogenierten oder nicht-halogenierten Kohlenwasserstoffen aufgeschäumt werden. Ganz besonders vorteilhafte Schäume sind durch Aufschäumen der erfindungsgemäßen Zubereitungen mit Kohlendioxid, Sauerstoff, Druckluft, Helium, Krypton, Xenon, Radon, Argon und/oder Stickstoff (sowohl einzeln als in beliebigen Mischungen miteinander) erhältlich.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Ethylhexylglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A, welches sich durch die folgende Strukturformel auszeichnet:

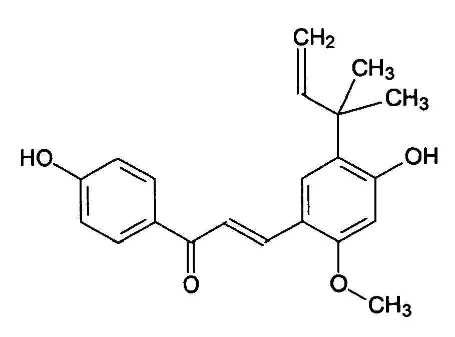

Licocalchon kann vorteilhaft auch als Bestandteil von pflanzlichen Extrakten, insbesondere von wäßrigen *Radix Glycyrrhizae inflatae,* eingesetzt werden.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem Extrakt aus *Radix Glycyrrhizae inflatae* enthalten, jeweils bezogen auf das Gesamtgewicht der Zubereitung.

Ganz besonders vorteilhaft ist es, von einem Extrakt auszugehen, der unter der Bezeichnung Polyol Soluble Licorice Extract PU (INCl-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcone A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Methylpropandiol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

Es wurden verschiedene Muster hinsichtlich ihrer Blasenzahl, Blasengröße, Blasenanteil und Blasenumfang untersucht.

| | **Muster A** | **Muster B** |
|---|---|---|
| | (Vergleichsmuster) | |
| | Gew.-% | Gew.% |
| Stearinsäure | 2,00 | 2,00 |
| Cetylalkohol | 2,00 | 2,00 |
| PEG-40 Stearat | 2,00 | 2,00 |
| Sorbitan Stearat | 0,75 | 0,75 |
| Cetyl Ricinolate | 1,00 | 1,00 |
| Butylenglycol Caprylat/Caprat | 8,00 | 8,00 |
| ***Ethylhexyl Triazin*** | | ***2,00*** |
| Cyclomethicon | 0,50 | 0,50 |
| Glycerin | 5,00 | 5,00 |
| Parfüm, Konservierungsmittel, Farbstoffe, usw. | q.s. | q.s. |
| Natriumydroxid | q.s | q.s |
| Wasser | ad 100 | ad 100 |

pH-Wert eingestellt auf 5,0-6,0
Zur Herstellung des Schaums werden 96 Vol.-% der Emulsion mit 4 Vol.-% Isobutan/Propan/Butan aufgeschäumt.

Die mikroskopische Schaumuntersuchung zeigte die folgenden Ergebnisse:

| | **Muster A** | **Muster B** |
|---|---|---|
| | (Vergleichsmuster) | |
| Blasenanzahl | 954 | 2143 |
| Blasenanteil (%) | 48 | 45 |
| mittlere Blasengrösse (mm²) | 0,02 | 0,01 |
| mittlerer Umfang (mm) | 0,45 | 0,3 |
| mittlerer Abstand | 16,8 | 11,4 |

Es ist deutlich zu erkennen, dass die Blasengröße im Muster B kleiner ist, wohingegen die Blasenanzahl im untersuchten Bildausschnitt (d. h. im gleichen Volumen) deutlich größer ist als in Muster A. Das bedeutet, daß die Zubereitung im Sinne der vorliegenden Erfindung deutlich mehr und deutlich kleinere Blasen aufweist als die Zubereitung des Standes der Technik. Ferner ist der Anteil der Blasen (der Gasanteil der Zubereitung) geringer, man erhält also im Verhältnis bei gleichem Volumen mehr Emulsion. Daraus lässt sich u. a. die kompaktere, pflegendere Textur der Zubereitung im Sinne der vorliegenden Erfindung erklären.

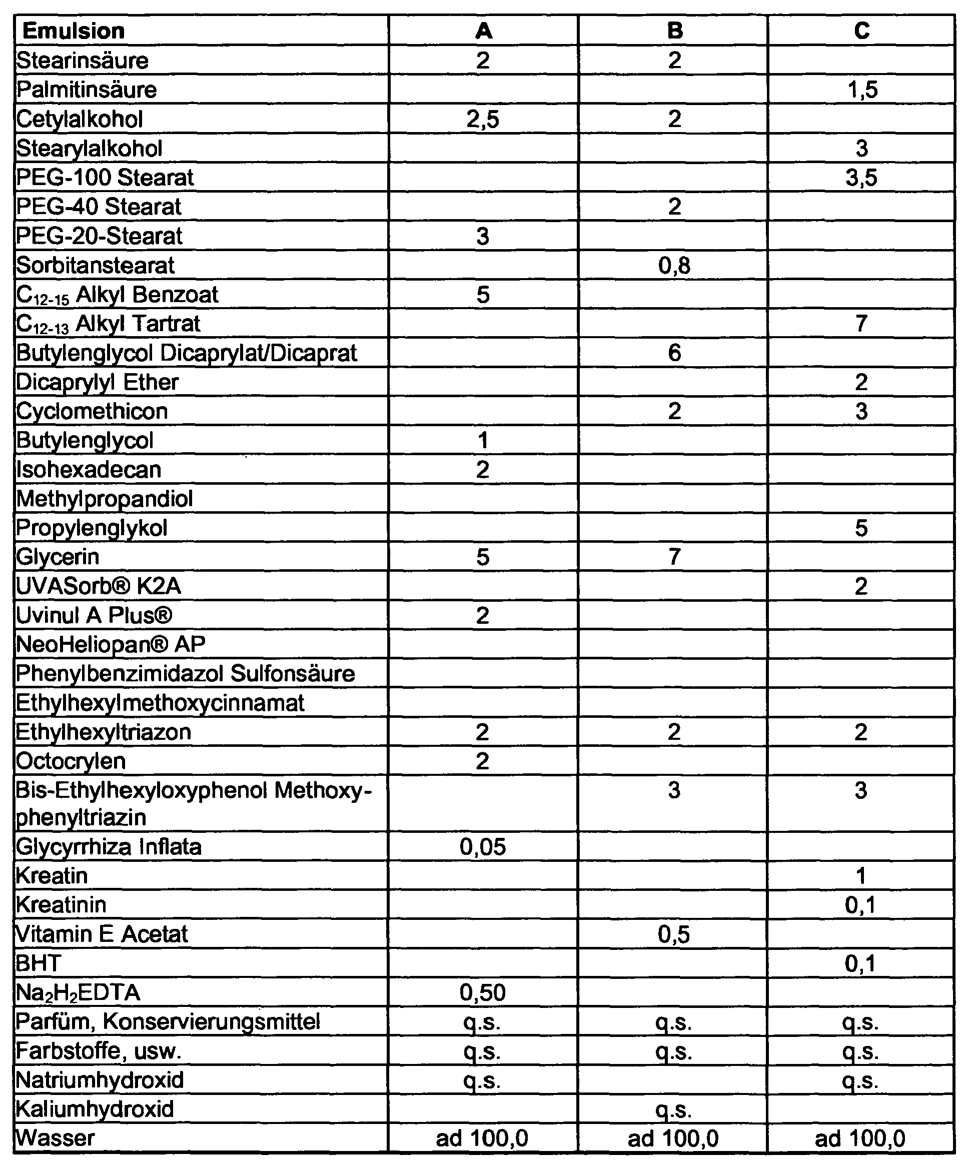

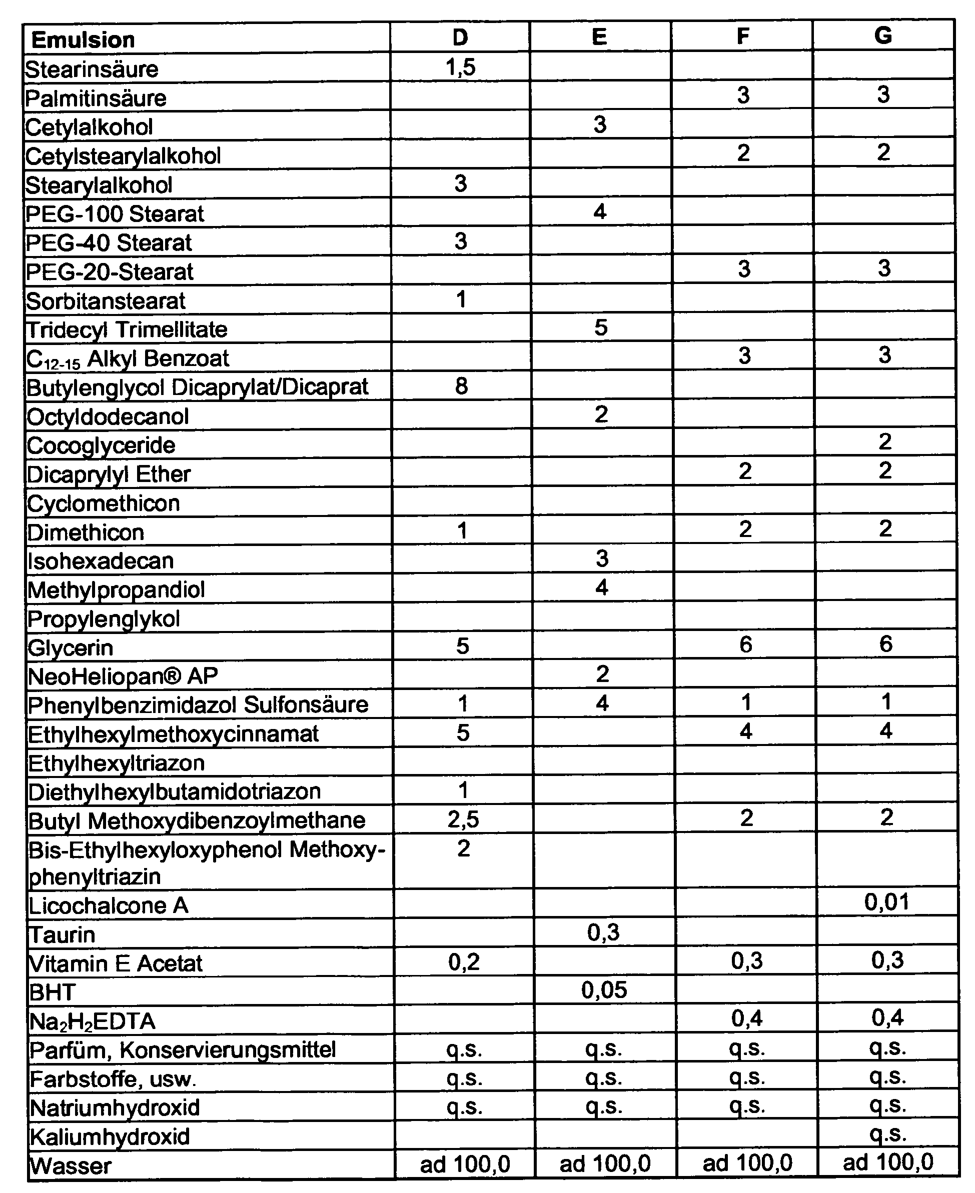

Zur Herstellung des (Aerosol-) Schaums werden jeweils 80 - 97 Vol.-% der Emulsion (A - G) mit 3 - 20 Vol.-% eines geigneten Gases (z. B. Propan/ Butan, Druckluft, Stickstoff) aufgeschäumt.

## Patentansprüche

1. Verwendung einer oder mehrerer UV-Filtersubstanzen zur Schaumverstärkung selbstschäumender, schaumförmiger, nachschäumender oder schäumbarer kosmetischer und dermatologischer Zubereitungen, welche mindestens eine polare Ölkomponente enthalten und wobei die UV-Filtersubstanz(en) (eine oder mehrere Verbindungen) aus der Gruppe:
• 2,4,6-Tris-[anilino-(p-carbo-2'-ethyl-1'-hexyloxy)]-1,3,5-triazin,
• 2-Ethylhexyl-4-methoxycinnamat,
• 4-(tert.-Butyl)-4'-methoxydibenzoylmethan,
• 2,4-Bis-{[4-(2-Ethyl-hexyloxy)-2-hydroxy]-phenyl}-6-(4-methoxyphenyl)-1,3,5-triazin und
• 2-Phenylbenzimidazol-5-sulfonsäure und/oder ihre Salze
gewählt wird.

2. Verwendung einer oder mehrerer UV-Filtersubstanzen zur Verringerung der Blasengröße selbstschäumender, schaumförmiger, nachschäumender oder schäumbarer kosmetischer und dermatologischer Zubereitungen.

3. Verwendung nach Anspruch 2, **dadurch gekennzeichnet, dass** die Zubereitung mindestens eine polare Ölkomponente enthält.

4. Verwendung nach einem der Ansprüche 2 oder 3, **dadurch gekennzeichnet, dass** die Zubereitungen eine mittlere Blasengröße von 0,005 bis 0,5 mm² haben.

## Claims

1. Use of one or more UV filter substances for the foam-boosting of self-foaming, foam-like, after-foaming or foamable cosmetic and dermatological preparations which comprise at least one polar oil component and where the UV filter substance(s) (one or more compounds) is chosen from the group:
• 2,4,6-tris[anilino(p-carbo-2'-ethyl-1'-hexyloxy)]-1,3,5-triazine,
• 2-ethylhexyl 4-methoxycinnamate,
• 4-(tert-butyl)-4'-methoxydibenzoylmethane,
• 2,4-bis{[4-(2-ethylhexyloxy)-2-hydroxy]phenyl}-6-(4-methoxyphenyl)-1,3,5-triazine and
• 2-phenylbenzimidazole-5-sulphonic acid and/or its salts.

2. Use of one or more UV filter substances for reducing the bubble size of self-foaming, foam-like, after-foaming or foamable cosmetic and dermatological preparations.

3. Use according to Claim 2, **characterized in that** the preparation comprises at least one polar oil component.

4. Use according to one of Claims 2 or 3, **characterized in that** the preparations have an average bubble size of from 0.005 to 0.5 mm².

## Revendications

1. Utilisation d'une ou de plusieurs substances filtre UV en vue du renforcement de mousse de préparations cosmétiques et dermatologiques auto-moussantes, sous forme de mousse, post-moussantes ou susceptibles d'être transformées en mousses, qui contiennent au moins un composant d'huile polaire et où la ou les substances filtre UV (un ou plusieurs composés) sont sélectionnées parmi le groupe:
• de la 2,4,6-tris-[anilino-(p-carbo-2'-éthyl-1'-hexyloxy)]-1,3,5-triazine,
• du 4-méthoxycinnamate de 2-éthylhexyle,
• du 4-(tert.butyl)-4'-méthoxydibenzoylméthane,
• de la 2,4-bis-{[4-(2-éthyl-hexyloxy)-2-hydroxy]-phényl}-6-(4-méthoxyphényl)-1,3,5-triazine et
• de l'acide 2-phénylbenzimidazol-5-sulfonique et/ou de leurs sels.

2. Utilisation d'une ou de plusieurs substances filtre UV en vue de l'amoindrissement de la taille des bulles de préparations cosmétiques et dermatologiques auto-moussantes, sous forme de mousse, post-moussantes ou susceptibles d'être transformées en mousses.

3. Utilisation selon la revendication 2, **caractérisée en ce que** la préparation contient au moins un composant d'huile polaire.

4. Utilisation selon l'une quelconque des revendications 2 ou 3, **caractérisée en ce que** les préparations ont une taille de bulle moyenne de 0,005 à 0,5 mm².
